# EUROPEAN PATENT APPLICATION

(11) **EP 4 176 920 A1**
(43) Date of publication of application: **10.05.2023**
(21) Application number: 20943142.8
(22) Date of filing: 03.07.2020
(51) Int. Cl.: A61M 25/09

(54) **GUIDE WIRE**

(71) Applicant: Asahi Intecc Co., Ltd., Seto-shi, Aichi 489-0071 (JP)
(72) Inventor: YAMADA Masayoshi, Seto-shi, Aichi 489-0071 (JP); SAWAI Akira, Seto-shi, Aichi 489-0071 (JP)
(74) Representative: Prinz & Partner mbB
(86) International application number: PCT/JP2020/026136
(87) International publication number: WO 2022/003931

(57) **Abstract**

A guide wire includes: a shaft body having a specific portion containing a radiopaque material; and a distal helical body formed into a spiral shape, which contains a radiopaque material and has at least a part located on a more distal end side relative to the shaft body. The guide wire includes a first radiopaque portion as a part of the guide wire in a longitudinal direction of the guide wire, which includes at least a part of the distal helical body. The guide wire further includes a second radiopaque portion as another part of the guide wire in the longitudinal direction of the guide wire, which is located on a more proximal end side relative to the first radiopaque portion, includes at least a part of the specific portion of the shaft body, and has a radiopacity different from that of the first radiopaque portion.

## Description

### TECHNICAL FIELD

The technology disclosed in the present specification relates to a guide wire to be inserted into a blood vessel or the like.

### BACKGROUND ART

Methods using a catheter are widely implemented as a method for treating or examining a constricted part or occluded part (hereinafter referred to as "lesion") in a blood vessel or the like. Typically, guide wires are used to guide a catheter to a lesion in a blood vessel or the like.

Guide wires include an elongated shaft body, and a distal helical body (e.g., coil body) formed into a spiral shape and having at least a part located on a more distal end side relative to the shaft body. The distal helical body is configured to contain a radiopaque material such as platinum (e.g., see PATENT DOCUMENT 1).

### CITATION LIST

### Patent Literature

Japanese Patent Laid-Open No. 2006-255396

### SUMMARY OF INVENTION

### TECHNICAL PROBLEM

In conventional guide wires, since a distal helical body contains a radiopaque material, the distal helical body can be visually recognized clearly by irradiating a living body with radiation from the outside of the living body while the guide wire is inserted into the living body. As a result, a distal end position of the guide wire can be reliably comprehended. However, in conventional guide wires, parts other than the distal helical body (e.g., the shaft body) are free from radiopaque materials, and therefore the other parts cannot be visually recognized clearly even under irradiation. Thus, with conventional guide wires, it is difficult to comprehend a blood vessel path by the guide wire itself without using a contrast medium or the like. Thus, with conventional guide wires, it is difficult to comprehend the blood vessel path by the guide wire itself while reliably comprehending the distal end position of the guide wire, leaving a room for improvement in terms of convenience.

The present specification discloses a technique capable of solving the above-described problem.

### SOLUTION TO PROBLEM

A technique disclosed in the present specification can be realized as the following aspects, for example.
(1) The guide wire disclosed in the present specification includes: a shaft body having a specific portion containing a radiopaque material; and a distal helical body formed into a spiral shape, which contains a radiopaque material and has at least a part located on a more distal end side relative to the shaft body. The guide wire has: a first radiopaque portion as a part of the guide wire in the longitudinal direction of the guide wire, which includes at least a part of the distal helical body; and a second radiopaque portion as another part of the guide wire in the longitudinal direction of the guide wire, which is located on a more proximal end side relative to the first radiopaque portion, includes at least a part of the specific portion of the shaft body, and has a radiopacity different from that of the first radiopaque portion.
   In this way, the guide wire according to this aspect has the first radiopaque portion as a part of the guide wire in the longitudinal direction, which includes at least a part of the distal helical body. Thereby, among the respective portions of the guide wire in the longitudinal direction, the first radiopaque portion can be visually recognized clearly by irradiating a living body with radiation from the outside of the living body while the guide wire is inserted into the living body. Thus, the guide wire according to this aspect makes it possible to reliably comprehend the distal end position of the guide wire. The guide wire according to this aspect also has a second radiopaque portion as another part of the guide wire in the longitudinal direction, which is located on a more proximal end side relative to the first radiopaque portion and includes at least a part of the specific portion containing a radiopaque material in the shaft body. Thereby, among the respective portions of the guide wire in the longitudinal direction, the second radiopaque portion can also be visually recognized clearly by irradiating a living body with radiation from the outside of the living body while the guide wire is inserted into the living body. In the guide wire according to this aspect, the second radiopaque portion has a radiopacity different from that of the first radiopaque portion. Thereby, the second radiopaque portion can be visually recognized clearly and distinguishably from the first radiopaque portion by irradiating a living body with radiation from the outside of the living body while the guide wire is inserted into the living body. Thus, the guide wire according to this aspect makes it possible to comprehend not only the position of the distal end portion of the guide wire but also a position of a more proximal end portion of the guide wire (the second radiopaque portion). This makes it possible for an operator such as a surgeon to comprehend a blood vessel path by the guide wire itself without using a contrast medium by observing the shape of the guide wire curved so as to follow the blood vessel path under irradiation. As described above, the guide wire according to this aspect makes it possible to comprehend the blood vessel path by the guide wire itself while reliably comprehending the distal end position of the guide wire, to improve the convenience of the guide wire.
(2) The guide wire according to the above aspect may be configured such that the first radiopaque portion is more radiopaque than the second radiopaque portion. The guide wire according to this aspect makes it possible to improve the visibility for the distal end portion of the guide wire that requires the highest visibility while allowing comprehension of the blood vessel path by the guide wire itself.
(3) The guide wire according to the above aspects may be configured such that the second radiopaque portion has a length larger than of the first radiopaque portion in the longitudinal direction of the guide wire. The guide wire according to this aspect makes it possible to comprehend the blood vessel path over a wider range by the guide wire itself while reliably comprehending the distal end position of the guide wire, to further improve the convenience of the guide wire.
(4) The guide wire according to the above aspects may be configured so as to further have a radiation-transmissible portion as another part of the guide wire in the longitudinal direction of the guide wire, which is located between the first radiopaque portion and the second radiopaque portion and free from radiopaque materials. In the guide wire according to this aspect, the first radiopaque portion and the second radiopaque portion can be more clearly distinguished from each other by the presence of the radiation-transmissible portion free from radiopaque materials. Thus, the guide wire according to this aspect can more reliably achieve both comprehension of the distal end position of the guide wire by the presence of the first radiopaque portion and comprehension of the blood vessel path by the presence of the second radiopaque portion.
(5) The guide wire according to the above aspects may be configured to further have a third radiopaque portion as another part of the guide wire in the longitudinal direction of the guide wire, which is located between the first radiopaque portion and the second radiopaque portion, and contains a radiopaque material, in which the first radiopaque portion is more radiopaque than the second radiopaque portion, and the third radiopaque portion is more radiopaque than the first radiopaque portion. In the guide wire according to this aspect, the first radiopaque portion and the second radiopaque portion can be more clearly distinguished from each other by the presence of the third radiopaque portion that is the most radiopaque. Thus, the guide wire according to this aspect can more reliably achieve both comprehension of the distal end position of the guide wire by the presence of the first radiopaque portion and comprehension of the blood vessel path by the presence of the second radiopaque portion.
(6) The guide wire according to the above aspects may be configured such that the shaft body has: a perforated shaft having a hole that opens on the distal end of the perforated shaft and extends in the longitudinal direction of the perforated shaft; and a first core member accommodated in the hole and serving as the specific portion containing a radiopaque material. In the guide wire according to this aspect, the first core member as the specific portion containing a radiopaque material is accommodated in the hole in the perforated shaft, and an outer peripheral portion of the shaft body is composed of the perforated shaft. Thus, in the guide wire according to this aspect, the first core member makes it possible to dispose the second radiopaque portion in the guide wire and to avoid wear and damage of the first core member because the first core member is not exposed, and the perforated shaft makes it possible to secure properties required for the shaft body (e.g., torquability, pushability, durability).
(7) The guide wire according to the above aspects may be configured such that, within the hole formed in the perforated shaft, a portion on a more proximal end side relative to a portion accommodating the first core member is hollow. The guide wire according to this aspect can be constitutively simplified and lightened. In the guide wire according to this aspect, the portion on the more proximal end side relative to a portion accommodating the first core member within the hole is hollow, and since this hollow portion is free from radiopaque materials, the second radiopaque portion can be more clearly recognized.
(8) The guide wire according to the above aspects may be configured such that the shaft body further has a second core member accommodated in the portion on the more proximal end side relative to a portion accommodating the first core member within the hole formed in the perforated shaft, the second core member being free from radiopaque materials. In the guide wire according to this aspect, the second core member makes it possible to suppress deterioration of the performance (e.g., torquability and durability) of the shaft body due to use of the perforated shaft for constituting the shaft body. In the guide wire according to this aspect, the second core member free from radiopaque materials is accommodated in the portion on the more proximal end side relative to the portion accommodating the first core member within the hole, and since this portion accommodating the second core member is free from radiopaque materials, the second radiopaque portion can be more clearly recognized.
(9) The guide wire according to the above aspects may be configured such that the hole formed in the perforated shaft is a bottomed hole having a bottom face at a more distal position relative to the proximal end of the perforated shaft. The guide wire according to this aspect makes it possible to reduce the area having the hole in the perforated shaft, so that deterioration of the performance (e.g., torquability and durability) of the shaft body due to use of the perforated shaft for constituting the shaft body can be effectively suppressed.
(10) The guide wire according to the above aspects may be configured such that, in the second radiopaque portion, the perforated shaft has a stiffness higher than that of the first core member. In the guide wire according to this aspect, since the first core member generally contains a radiopaque material that has generally low stiffness, the performance (e.g., torquability and durability) of the shaft body tends to deteriorate. However, in the guide wire according to this aspect, the perforated shaft has the stiffness higher than that of the first core member in the second radiopaque portion. Thereby, the guide wire according to this aspect makes it possible to suppress deterioration of the performance (e.g., torquability and durability) of the shaft body due to use of the perforated shaft.
(11) The guide wire according to the above aspects may be configured as an atherectomy guide wire rotatably supporting a rotor disposed in an atherectomy catheter on the second radiopaque portion. As described above, in the guide wire according to this aspect, the second radiopaque portion can be visually recognized clearly and distinguishably from the first radiopaque portion by irradiating a living body with radiation from the outside of the living body while the guide wire is inserted into the living body. Thereby, it is possible to clearly comprehend that the rotor disposed in the atherectomy catheter is located on the second radiopaque portion of the guide wire, to prevent the rotor from coming into contact with the distal helical body. Since it is possible to determine whether or not the rotor rotates on the basis of the comprehension of the blood vessel path by the guide wire itself, for example, it is possible to prevent the rotor from rotating at a sharply curved position in the blood vessel, and therefore the safety can be improved.

The technology disclosed in the present specification may be realized in various forms, for example, in the form of a guide wire, a manufacturing method thereof, etc.

### BRIEF DESCRIPTION OF DRAWINGS

FIG. 1 is an explanatory diagram schematically illustrating a configuration of a guide wire 100 in a first embodiment.
FIG. 2 is an explanatory diagram schematically illustrating a configuration of a guide wire 100a in a second embodiment.
FIG. 3 is an explanatory diagram schematically illustrating a configuration of a guide wire 100b in a third embodiment.
FIG. 4 is an explanatory diagram schematically illustrating a configuration of a guide wire 100c in a fourth embodiment.
FIG. 5 is an explanatory diagram schematically illustrating a configuration of a guide wire 100d in a fifth embodiment.
FIG. 6 is an explanatory diagram schematically illustrating a configuration of a guide wire 100e in a sixth embodiment.

### EMBODIMENTS OF THE INVENTION

### A. First Embodiment:

### A-1. Basic Configuration of Guide Wire 100:

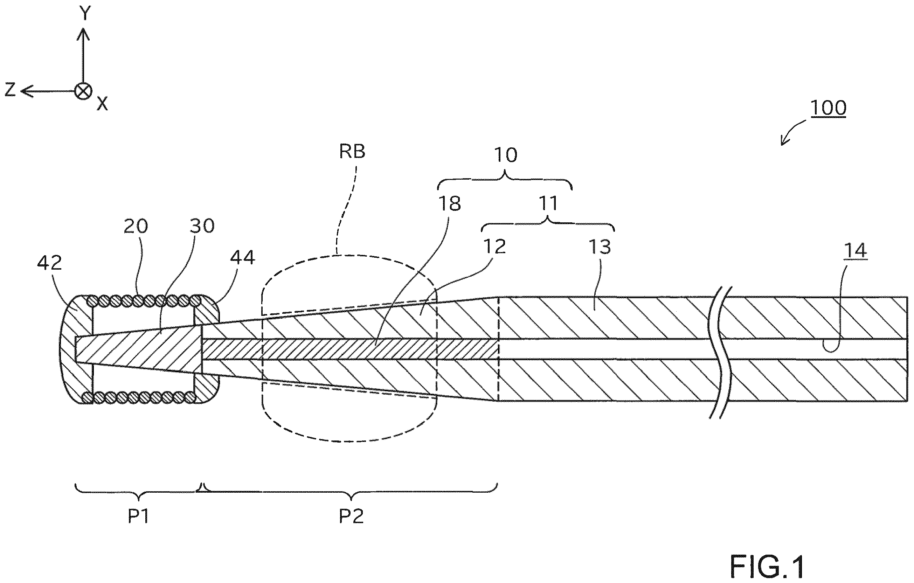

FIG. 1 is an explanatory diagram schematically illustrating a configuration of a guide wire 100 in the first embodiment. FIG. 1 illustrates a configuration of a longitudinal section (YZ section) of the guide wire 100 together with XYZ axes mutually orthogonal to each other for specifying directions. A part of the guide wire 100 is not illustrated in FIG. 1. In FIG. 1, a Z-axis positive direction side is a distal end side (far side) to be inserted into the body, and a Z-axis negative direction side is a proximal end side (near side) to be manipulated by a technician such as a surgeon. The same applies to the other drawings. In the present specification, with regard to the guide wire 100 and each component thereof, an end on the distal end side is referred to as "distal end", the distal end and its vicinity as "distal end portion", an end on the proximal end side as "proximal end", and the proximal end and its vicinity as "proximal end portion". FIG. 1 illustrates a state where the entire guide wire 100 has a straight shape substantially parallel to the Z-axis direction, but the guide wire 100 is flexible enough to be curved. In the present specification, for convenience of explanation, the guide wire 100 is assumed to be in the state illustrated in FIG. 1, and the Z-axis direction is referred to as a "longitudinal direction of the guide wire 100" or simply as a "longitudinal direction".

The guide wire 100 is an elongated medical device to be inserted into a blood vessel or the like, to guide a catheter into a lesion (constricted part or occluded part) in the blood vessel or the like. For example, the guide wire 100 has a total length of about 1000 mm to 4000 mm.

The guide wire 100 according to the first embodiment is an atherectomy guide wire for guiding an atherectomy catheter. The "atherectomy" refers to a treatment method using an atherectomy catheter equipped with a drill-shaped rotor RB into which micro-diamond has been embedded, in which a lesion (e.g., calcified lesion) is excised by rotating the rotor RB at a high speed (e.g., about 150,000 rpm). The rotor RB is rotatably supported on a second radiopaque portion P2 described later in the guide wire 100.

The guide wire 100 includes a shaft body 10, a distal helical body 20, a distal core member 30, a distal end-side joint part 42, and a proximal end-side joint part 44.

The shaft body 10 is an elongated member extending in the longitudinal direction. The shaft body 10 has a perforated shaft 11 and a radiopaque core member 18.

The perforated shaft 11 of the shaft body 10 is an elongated member extending in the longitudinal direction. The perforated shaft 11 is composed of a large diameter portion 13 having a substantially constant outer diameter, and a tapered portion 12 located on a more distal end side relative to the large diameter portion 13 and having an outer diameter gradually decreasing from a boundary position with the large diameter portion 13 toward the distal end side. An outer shape of a transverse section (XY section) at each position of the perforated shaft 11 may be any shape, such as a circular shape. An outer diameter of the large diameter portion 13 is e.g., about 0.15 to 0.89 mm, and an outer diameter of a smallest diameter portion of the tapered portion 12 is e.g., about 0.05 to 0.2 mm. A length of the tapered portion 12 in the longitudinal direction is e.g., about 30 to 500 mm.

The perforated shaft 11 has a hole 14 that opens on the distal end of the perforated shaft 11 and extends in the longitudinal direction. In the first embodiment, the hole 14 is a through-hole that penetrates from the distal end to the proximal end of the perforated shaft 11. A shape of the transverse section (XY section) at each position of the hole 14 may be any shape, such as a circular shape. An inner diameter of the hole 14 is substantially constant from the distal end to the proximal end of the perforated shaft 11, e.g., about 0.05 to 0.18 mm. The inner diameter of the hole 14 need not be constant from the distal end to the proximal end of the perforated shaft 11, and the inner diameters of the hole 14 at a plurality of positions in the longitudinal direction of the perforated shaft 11 may differ from each other.

As the material for forming the perforated shaft 11, known materials are used, such as metallic materials, more specifically, stainless steel (SUS302, SUS304, SUS316, etc.), and superelastic alloys such as Ni-Ti alloys. The entire perforated shaft 11 may be made of the same material, or each portion of the shaft 11 may be made of a different material from each other.

The radiopaque core member 18 is a rod-shaped member accommodated in the hole 14 formed in the perforated shaft 11. In the first embodiment, the radiopaque core member 18 is accommodated in a portion formed on the tapered portion 12 within the hole 14 formed in the perforated shaft 11. An outer shape of the transverse section (XY section) at each position of the radiopaque core member 18 may be any shape, such as a circular shape. An outer diameter of the radiopaque core member 18 is the same as or slightly smaller than the inner diameter of the hole 14 formed in the perforated shaft 11, e.g., about 0.05 to 0.18 mm. In the first embodiment, the radiopaque core member 18 is located throughout the portion formed on the tapered portion 12 within the hole 14 formed in the perforated shaft 11. Thus, a length of the radiopaque core member 18 in the longitudinal direction is substantially the same as the length of the tapered portion 12 of the perforated shaft 11, e.g., about 30 to 500 mm. The length of the radiopaque core member 18 in the longitudinal direction need not be substantially the same as the length of the tapered portion 12 of the perforated shaft 11, e.g., may be shorter than the length of the tapered portion 12. The portion formed on the large diameter portion 13 within the hole 14 formed in the perforated shaft 11 accommodates nothing. That means, the portion on the more proximal end side relative to the portion accommodating the radiopaque core member 18 within the hole 14 formed in the perforated shaft 11 is hollow.

The radiopaque core member 18 is configured to contain a radiopaque material (e.g., X-ray opaque material). The radiopaque material refers to a material having radiopacity, such as radiopaque metals and radiopaque resins. Examples of the radiopaque metals include, but are not limited to, platinum, gold, silver, tin, tungsten, bismuth, rhenium, tantalum, palladium, iridium, barium, and alloys thereof. As an example, the radiopaque core member 18 is made of a platinum alloy. The radiopaque core member 18 is described as an example of a first core member and a specific portion in claims.

The distal core member 30 is a rod-shaped member extending in the longitudinal direction. The distal core member 30 is located on a more distal end side relative to the radiopaque core member 18. In the first embodiment, the proximal end portion of the distal core member 30 is joined to the distal end portion of the perforated shaft 11 of the shaft body 10 e.g., by brazing or welding As the material for forming the distal core member 30, known materials are used, such as metallic materials, more specifically, stainless steel (SUS302, SUS304, SUS316, etc.), and superelastic alloys such as Ni-Ti alloys.

The distal helical body 20 is a member formed into a spiral shape. In the first embodiment, the distal helical body 20 is composed of a coil body formed into a hollow cylindrical shape by densely winding one strand into a spiral shape. At least a part (substantially entire part in the first embodiment) of the distal helical body 20 is located on a more distal end side relative to the shaft body 10. The distal helical body 20 is wound so as to cover at least a part (substantially entire part in the first embodiment) of the distal core member 30. A diameter of the strand constituting the distal helical body 20 is e.g., about 0.03 to 0.1 mm. An outer diameter of the distal helical body 20 is e.g., about 0.25 to 0.5 mm. A length of the distal helical body 20 in the longitudinal direction is e.g., about 15 to 40 mm. In the first embodiment, the length of the distal helical body 20 in the longitudinal direction is shorter than the length of the radiopaque core member 18 constituting the shaft body 10.

The distal helical body 20 is configured to contain a radiopaque material (e.g., X-ray opaque material). The radiopaque material contained in the distal helical body 20 may be the same as or different from the radiopaque material contained in the radiopaque core member 18 constituting the shaft body 10. As an example, the distal helical body 20 is made of a platinum alloy.

The distal end-side joint part 42 is a member that joins the distal end of the distal helical body 20 with the distal end of the distal core member 30. A distal end-side outer peripheral surface of the distal end-side joint part 42 is a smooth (e.g., substantially hemispherical face). The proximal end-side joint part 44 is a member that joins the proximal end of the distal helical body 20 with the distal end of the shaft body 10. As the materials constituting the distal end-side joint part 42 and the proximal end-side joint part 44, known materials are used, such as brazing materials (aluminum alloy braze, silver braze, gold braze, etc.), metal solders (Ag-Sn alloys, Au-Sn alloys, etc.), and adhesives (epoxy-based adhesives, etc.).

Since the guide wire 100 according to the first embodiment is configured as described above, the guide wire 100 has two radiopaque portions (a first radiopaque portion P1 and a second radiopaque portion P2) individually as a part of the guide wire 100 in the longitudinal direction (i.e. each part obtained by hypothetically dividing the guide wire 100 into a plurality of parts aligned in the longitudinal direction). The first radiopaque portion P1 includes at least a part of the distal helical body 20. In the first embodiment, the first radiopaque portion P1 is composed of the entire distal helical body 20 and the entire distal core member 30. Since the distal helical body 20 contains the radiopaque material, the first radiopaque portion P1 has radiopacity. The second radiopaque portion P2 is located on the more proximal end side relative to the first radiopaque portion P1 and includes at least a part of the radiopaque core member 18 of the shaft body 10. In the first embodiment, the second radiopaque portion P2 is composed of the entire radiopaque core member 18 and the tapered portion 12 of the perforated shaft 11.

In the guide wire 100 according to the first embodiment, the radiopacity of the first radiopaque portion P1 and the radiopacity of the second radiopaque portion P2 are different from each other. More specifically, the first radiopaque portion P1 is more radiopaque than the second radiopaque portion P2. The radiopacity means a level of the performance for preventing radiation transmission. In other words, the radiopacity means a level of the visibility under irradiation.

The above-described high/low relationship of the radiopacity between the first radiopaque portion P1 and the second radiopaque portion P2 can be established by adjusting the materials and shapes of the distal helical body 20 and the radiopaque core member 18. For example, the above-described high/low relationship of the radiopacity can be established by forming the distal helical body 20 and the radiopaque core member 18 using the same radiopaque material and by making the outer diameter of the distal helical body 20 or the diameter of the strand larger than the outer diameter of the radiopaque core member 18. Alternatively, the above-described high/low relationship of the radiopacity can be established by using, as the material of the distal helical body 20, a material that is more radiopaque than the material of the radiopaque core member 18.

The high/low relationship of the radiopacity between each portion of the guide wire 100 can be determined as follows. The guide wire 100 is irradiated with an X-ray using an X-ray apparatus under a prescribed output condition (60kV, 10W) to acquire an image of the guide wire 100 (8-bit monochrome image (black pixel value: 0, white pixel value: 255)). In the acquired image, a color of a portion with higher radiopacity in the guide wire 100 is closer to black, and a color of a portion with lower radiopacity is closer to white. An average value of each pixel expressing each portion of the guide wire 100 is calculated using an image processing software. An average pixel value Av1 of a part (hereinafter referred to as a "first portion") of the guide wire in the longitudinal direction, and an average pixel value Av2 of another part (hereinafter referred to as a "second portion") of the guide wire in the longitudinal direction are calculated (with the proviso that Av1 is greater than Av2). If a value of (Av1-Av2)/Av1 is 0.1 or greater, the radiopacity of the second portion of the guide wire is determined to be different from the radiopacity of the first portion of the guide wire (the second portion of the guide wire is more radiopaque than the first portion of the guide wire).

In the guide wire 100 according to the first embodiment, the length of the radiopaque core member 18 is larger than the length of the distal helical body 20 in the longitudinal direction. Thus, a length of the second radiopaque portion P2 is larger than a length of the first radiopaque portion P1 in the longitudinal direction. In the first embodiment, the perforated shaft 11 has a stiffness higher than that of the radiopaque core member 18 in the second radiopaque portion P2. This high/low relationship of stiffness can be established by adjusting the materials and shapes of the radiopaque core member 18 and the perforated shaft 11. For example, the above-described high/low relationship can be established by forming the perforated shaft 11 using a material with a relatively high elastic modulus or by making the outer diameter of the perforated shaft 11 relatively large.

### A-2. Manufacturing Method of Guide Wire 100:

For example, the guide wire 100 according to the first embodiment can be manufactured as follows. First, for example, a stainless steel hypotube is prepared and subjected to tapering or the like to produce the perforated shaft 11 composed of the tapered portion 12 and the large diameter portion 13. The rod-shaped radiopaque core member 18 is prepared using a radiopaque material. For the purpose of inserting the radiopaque core member 18 smoothly into the hole 14 of the perforated shaft 11, the outer diameter of the radiopaque core member 18 may be made slightly smaller than the inner diameter of the hole 14 of the perforated shaft 11. Subsequently, the radiopaque core member 18 is inserted into the hole 14 of the perforated shaft 11 from the distal end side of the perforated shaft 11. At this time, the entire radiopaque core member 18 is accommodated in the hole 14 of the perforated shaft 11. As a result, the shaft body 10 having the perforated shaft 11 and the radiopaque core member 18 is prepared.

Next, the distal core member 30 is prepared, and the proximal end portion of the distal core member 30 is joined to the distal end portion of the perforated shaft 11 of the shaft body 10 e.g., by brazing or welding. Subsequently, the distal helical body 20 is prepared and joined to the distal core member 30 and the shaft body 10. Specifically, the distal end-side joint part 42 that joins the distal helical body 20 with the distal core member 30, and the proximal end-side joint part 44 that joins the distal helical body 20 with the shaft body 10 are formed e.g., by brazing or the like. Primarily by the above-described method, the guide wire 100 according to the first embodiment is manufactured.

### A-3. Effect of First Embodiment:

As explained above, the guide wire 100 according to the first embodiment includes the shaft body 10 and the distal helical body 20. The shaft body 10 has a radiopaque core member 18 (specific portion) containing a radiopaque material. The distal helical body 20 contains a radiopaque material and is formed into a spiral shape. At least a part of the distal helical body 20 is located on a more distal end side relative to the shaft body 10. The guide wire 100 according to the first embodiment has the first radiopaque portion P1 and the second radiopaque portion P2, each of which is a part of the guide wire 100 in the longitudinal direction. The first radiopaque portion P1 is a radiopaque portion including at least a part of the distal helical body 20. The second radiopaque portion P2 is a radiopaque portion located on a more proximal end side relative to the first radiopaque portion P1 and including at least a part of the radiopaque core member 18 of the shaft body 10. The second radiopaque portion P2 has a radiopacity different from the radiopacity of the first radiopaque portion P1.

In this way, the guide wire 100 according to the first embodiment has the first radiopaque portion P1 as a part of the guide wire 100 in the longitudinal direction, which includes at least a part of the distal helical body 20. Thereby, among the respective portions of the guide wire 100 in the longitudinal direction, the first radiopaque portion P1 can be visually recognized clearly by irradiating a living body with radiation from the outside of the living body while the guide wire 100 is inserted into the living body. Thus, the guide wire 100 according to the first embodiment makes it possible to reliably comprehend the distal end position of the guide wire 100. The guide wire 100 according to the first embodiment has the second radiopaque portion P2 as another part of the guide wire 100 in the longitudinal direction, which is located on a more proximal end side relative to the first radiopaque portion P1 and includes at least a part of the radiopaque core member 18 of the shaft body 10. Thereby, among the respective portions of the guide wire 100 in the longitudinal direction, the second radiopaque portion P2 can also be visually recognized clearly by irradiating a living body with radiation from the outside of the living body while the guide wire 100 is inserted into the living body. In the guide wire 100 according to the first embodiment, the second radiopaque portion P2 has a radiopacity different from the radiopacity of the first radiopaque portion P1. Thereby, the second radiopaque portion P2 can be visually recognized distinguishably from the first radiopaque portion P1 by irradiating a living body with radiation from the outside of the living body while the guide wire 100 is inserted into the living body. Thus, the guide wire 100 according to the first embodiment makes it possible to comprehend not only the distal end position of the guide wire 100 but also a position of a more proximal end portion (second radiopaque portion P2). This makes it possible for an operator such as a surgeon to comprehend the blood vessel path by the guide wire 100 itself without using a contrast medium by observing the shape of the guide wire 100 curved so as to follow the blood vessel path under irradiation. As described above, the guide wire 100 according to the first embodiment makes it possible to comprehend the blood vessel path by the guide wire 100 itself while reliably comprehending the distal end position of the guide wire 100, to improve the convenience of the guide wire 100.

In the guide wire 100 according to the first embodiment, the first radiopaque portion P1 is more radiopaque than the second radiopaque portion P2. Thereby, the guide wire 100 according to the first embodiment makes it possible to improve the visibility for the distal end portion of the guide wire 100 that requires the highest visibility while allowing comprehension of the blood vessel path by the guide wire 100 itself.

In the guide wire 100 according to the first embodiment, the length of the second radiopaque portion P2 is larger than the length of the first radiopaque portion P1 in the longitudinal direction. Thus, the guide wire 100 according to the first embodiment makes it possible to comprehend the blood vessel path over a wider range by the guide wire 100 itself while reliably comprehending the distal end position of the guide wire 100, to further improve the convenience of the guide wire 100.

In the guide wire 100 according to the first embodiment, the shaft body 10 has the perforated shaft 11 and the radiopaque core member 18. The perforated shaft 11 has the hole 14 that opens on the distal end of the perforated shaft 11 and extends in the longitudinal direction of the perforated shaft 11. The radiopaque core member 18 is accommodated in the hole 14 of the perforated shaft 11 and contains a radiopaque material. As described above, in the guide wire 100 according to the first embodiment, the radiopaque core member 18 is accommodated in the hole 14 of the perforated shaft 11, and an outer peripheral portion of the shaft body 10 is composed of the perforated shaft 11. Thus, in the guide wire 100 according to the first embodiment, the radiopaque core member 18 makes it possible to dispose the second radiopaque portion P2 in the guide wire 100 and to avoid wear and damage of the radiopaque core member 18 because the radiopaque core member 18 is not exposed, and the perforated shaft 11 makes it possible to secure properties required for the shaft body 10 (e.g., torquability, pushability, durability).

In the guide wire 100 according to the first embodiment, the portion on the more proximal end side relative to the portion accommodating the radiopaque core member 18 within the hole 14 formed in the perforated shaft 11 is hollow. Thus, the guide wire 100 according to the first embodiment can be constitutively simplified and lightened. In the guide wire 100 according to the first embodiment, the portion on the more proximal end side relative to the portion accommodating the radiopaque core member 18 within the hole 14 is hollow, and since this hollow portion is free from radiopaque materials, the second radiopaque portion P2 can be more clearly recognized.

In the guide wire 100 according to the first embodiment, the perforated shaft 11 has a stiffness higher than that of the radiopaque core member 18 in the second radiopaque portion P2. As described above, in the guide wire 100 according to the first embodiment, since the radiopaque core member 18 generally contains a radiopaque material with low stiffness, the performance (e.g., torquability and durability) of the shaft body 10 tends to deteriorate. However, in the guide wire 100 according to the first embodiment, the perforated shaft 11 has the stiffness higher than that of the radiopaque core member 18 in the second radiopaque portion P2. Thereby, the guide wire 100 according to the first embodiment makes it possible to suppress deterioration of the performance (e.g., torquability and durability) of the shaft body 10 due to use of the perforated shaft 11.

The guide wire 100 according to the first embodiment is an atherectomy guide wire that rotatably supports the rotor RB disposed in an atherectomy catheter on the second radiopaque portion P2. As described above, in the guide wire 100 according to the first embodiment, the second radiopaque portion P2 can be visually recognized clearly and distinguishably from the first radiopaque portion P1 by irradiating a living body with radiation from the outside of the living body while the guide wire 100 is inserted into the living body. Thereby, it is possible to clearly comprehend that the rotor RB disposed in the atherectomy catheter is located on the second radiopaque portion P2 of the guide wire 100, to prevent the rotor RB from coming into contact with the distal helical body 20. Since it is possible to determine whether or not the rotor RB rotates on the basis of the comprehension of the blood vessel path by the guide wire 100 itself, for example, it is possible to prevent the rotor RB from rotating at a sharply curved position in the blood vessel, and therefore the safety can be improved.

The guide wire 100 according to the first embodiment is configured such that the perforated shaft 11 has the stiffness higher than that of the radiopaque core member 18 in the second radiopaque portion P2 as described above, and therefore the stiffness decreases inward in the radial direction of the shaft body 10. Thus, in the guide wire 100 according to the first embodiment, when the rotor RB is slid at a curved position of a blood vessel, breakage of the guide wire 100 due to the sliding can be prevented (durability of the guide wire 100 against the sliding can be improved).

### B. Second Embodiment:

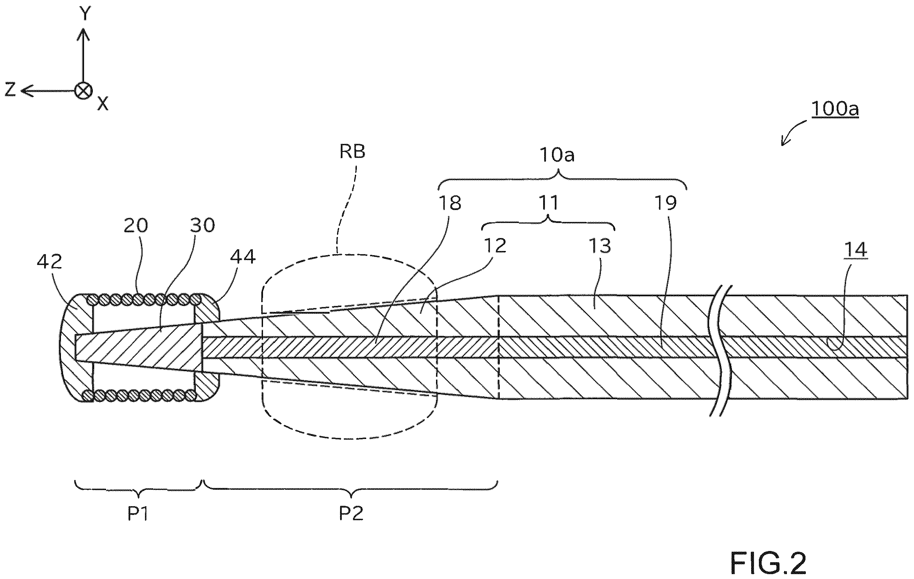

FIG. 2 is an explanatory diagram schematically illustrating a configuration of a guide wire 100a according to the second embodiment. In the description below, components of the guide wire 100a according to the second embodiment, which are the same as the above-described components of the guide wire 100 according to the first embodiment, are given the same reference signs as in the first embodiment so as to omit the description of the same components in the second embodiment as appropriate.

The guide wire 100a according to the second embodiment includes a shaft body 10a having a structure different from that of the guide wire 100 according to the first embodiment. Specifically, in the guide wire 100a according to the second embodiment, the shaft body 10a has a radiation-transmissible core member 19 free from radiopaque materials.

The radiation-transmissible core member 19 is a rod-shaped member accommodated within the hole 14 formed in the perforated shaft 11. In the second embodiment, the radiation-transmissible core member 19 is accommodated in a portion formed on the large diameter portion 13 within the hole 14 formed in the perforated shaft 11. In other words, the radiation-transmissible core member 19 is accommodated in a portion on a more proximal end side relative to the portion accommodating the radiopaque core member 18 within the hole 14 formed in the perforated shaft 11. An outer shape of the transverse section (XY section) at each position of the radiation-transmissible core member 19 may be any shape, such as a circular shape. An outer diameter of the radiation-transmissible core member 19 is the same as or slightly smaller than the inner diameter of the hole 14 formed in the perforated shaft 11, e.g., about 0.05 to 0.18 mm. In the second embodiment, the radiation-transmissible core member 19 is located throughout the portion formed on the large diameter portion 13 within the hole 14 formed in the perforated shaft 11. For this reason, in the longitudinal direction, the radiation-transmissible core member 19 and the radiopaque core member 18 are in contact with each other, between which there is no gap.

For example, the shaft body 10a as configured above can be manufactured e.g., by inserting the radiation-transmissible core member 19 into the hole 14 in the perforated shaft 11 from the proximal end side of the perforated shaft 11 composed of a stainless steel hypotube and by inserting the radiopaque core member 18 into the hole 14 from the distal end side of the perforated shaft 11.

For example, the radiation-transmissible core member 19 is made of a metallic material, more specifically, stainless steel (SUS302, SUS304, SUS316, etc.), superelastic alloys such as Ni-Ti alloys, or the like. The radiation-transmissible core member 19 is an example of a second core member in claims.

Since the guide wire 100a according to the second embodiment has the same structure as the above-described structure of the guide wire 100 according to the first embodiment, the guide wire 100a exhibits the same effect as the above-described effect exhibited by the guide wire 100 according to the first embodiment. Moreover, in the guide wire 100a according to the second embodiment, a portion on a more proximal end side relative to the portion accommodating the radiopaque core member 18 within the hole 14 formed in the perforated shaft 11 is not hollow. That means, in the guide wire 100a according to the second embodiment, the shaft body 10 further has the radiation-transmissible core member 19 free from radiopaque materials, which is accommodated in a portion on a more proximal end side relative to the portion accommodating the radiopaque core member 18 within the hole 14 formed in the perforated shaft 11. Thereby, in the guide wire 100a according to the second embodiment, the radiation-transmissible core member 19 makes it possible to suppress deterioration of the performance (e.g., torquability and durability) of the shaft body 10a due to use of the perforated shaft 11 for constituting the shaft body 10a. In the guide wire 100a according to the second embodiment, the radiation-transmissible core member 19 free from radiopaque materials is accommodated in the portion on the more proximal end side relative to the portion accommodating the radiopaque core member 18 within the hole 14, and since this portion accommodating the radiation-transmissible core member 19 is free from radiopaque materials, the second radiopaque portion P2 can be more clearly recognized.

### C. Third Embodiment:

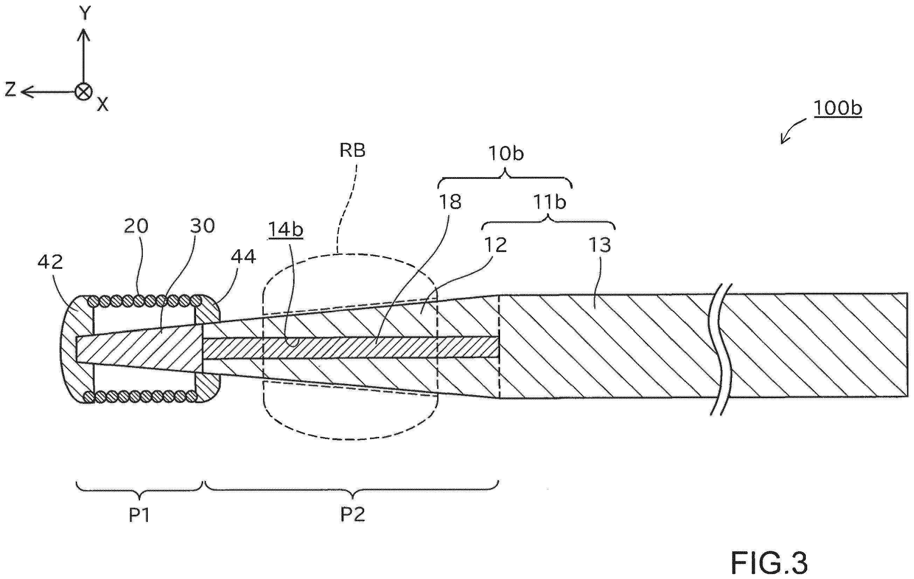

FIG. 3 is an explanatory diagram schematically illustrating a configuration of a guide wire 100b according to the third embodiment. In the description below, components of the guide wire 100b according to the third embodiment, which are the same as the above-described components of the guide wire 100 according to the first embodiment are given the same reference signs as in the first embodiment so as to omit the description of the same components in the third embodiment as appropriate.

The guide wire 100b according to the third embodiment includes a shaft body 10b having a structure different from that of the guide wire 100 according to the first embodiment. Specifically, in the guide wire 100b according to the third embodiment, a hole 14b formed in a perforated shaft 11b of the shaft body 10b is a bottomed hole that does not penetrate the proximal end of the perforated shaft 11b and has a bottom face at a more distal position relative to the proximal end of the perforated shaft 11b. In the third embodiment, the bottom face of the hole 14b formed in the perforated shaft 11b is located at a boundary between the tapered portion 12 and the large diameter portion 13. In the third embodiment, the radiopaque core member 18 is located throughout the hole 14b formed in the perforated shaft 11b.

The shaft body 10b as configured above can be manufactured e.g., by perforating a distal end side of a stainless steel wire to prepare the perforated shaft 11 having the bottomed hole 14b, and by inserting the radiopaque core member 18 into the hole 14b of the perforated shaft 11 from the distal end side of the perforated shaft 11.

Since the guide wire 100b according to the third embodiment has the same structure as the above-described structure of the guide wire 100 according to the first embodiment, the guide wire 100b exhibits the same effect as the above-described effect exhibited by the guide wire 100 according to the first embodiment. Moreover, in the guide wire 100b according to the third embodiment, the hole 14b formed in the perforated shaft 11b is a bottomed hole having a bottom face at a more distal position relative to the proximal end of the perforated shaft 11b. Thus, the guide wire 100b according to the third embodiment makes it possible to reduce the area having the hole 14b in the perforated shaft 11b, so that deterioration of the performance (e.g., torquability and durability) of the shaft body 10b due to use of the perforated shaft 11b for constituting the shaft body 10b can be effectively suppressed. In the guide wire 100b according to the third embodiment, the more proximal end side relative to the portion where the radiopaque core member 18 is located is free from radiopaque materials, and therefore the second radiopaque portion P2 can be more clearly recognized.

### D. Fourth Embodiment:

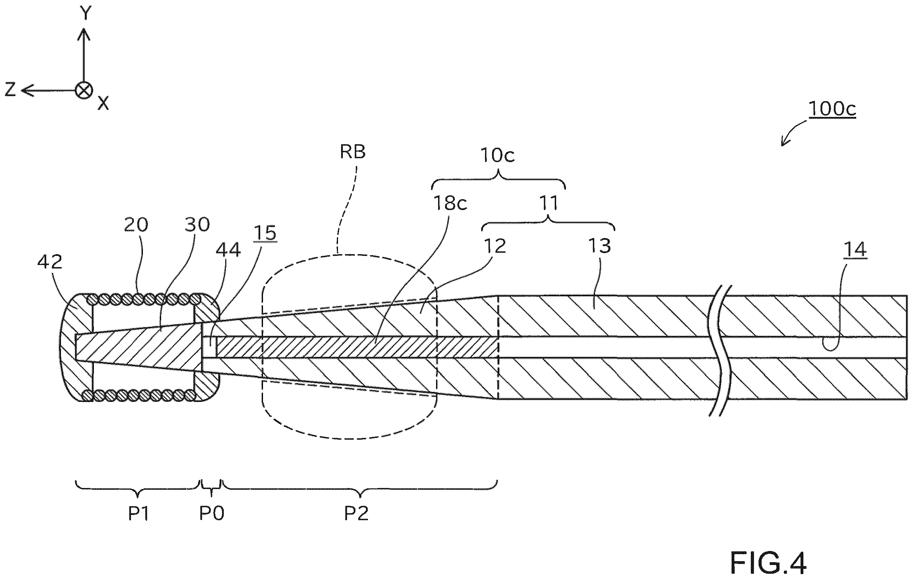

FIG. 4 is an explanatory diagram schematically illustrating a configuration of a guide wire 100c according to the fourth embodiment. In the description below, components of the guide wire 100c according to the fourth embodiment, which are the same as the above-described components of the guide wire 100 according to the first embodiment are given the same reference signs as in the first embodiment so as to omit the description of the same components in the fourth embodiment as appropriate.

The guide wire 100c according to the fourth embodiment includes a shaft body 10c having a structure different from that of the guide wire 100 according to the first embodiment. Specifically, in the guide wire 100c according to the fourth embodiment, a distal end of a radiopaque core member 18c constituting the shaft body 10c is located on a more proximal end side relative to the distal end of the perforated shaft 11. As a result, there is a gap 15 between the radiopaque core member 18c and the distal core member 30 in the longitudinal direction.

Since the guide wire 100c according to the fourth embodiment is configured as described above, the second radiopaque portion P2 is composed of the radiopaque core member 18c and a part on the proximal end side of the tapered portion 12 of the perforated shaft 11. Thus, the guide wire 100c according to the fourth embodiment has a radiation-transmissible portion P0 as another part of the guide wire 100c in the longitudinal direction, which is located between the first radiopaque portion P1 and the second radiopaque portion P2 and free from radiopaque materials. In the fourth embodiment, the radiation-transmissible portion P0 is composed of a frontmost end portion (where the radiopaque core member 18c is not located) of the perforated shaft 11 of the shaft body 10c.

For example, the shaft body 10c as configured above can be manufactured e.g., by a process in which, when the radiopaque core member 18c is inserted into the perforated shaft 11 composed of a stainless steel hypotube from the distal end side of the perforated shaft 11, and in a state that the position of the distal end of the radiopaque core member 18c is identical to the position of the distal end of the perforated shaft 11, the radiopaque core member 18c is further pushed in the perforated shaft 11.

Since the guide wire 100c according to the fourth embodiment has the same structure as the above-described structure of the guide wire 100 according to the first embodiment, the guide wire 100c exhibits the same effect as the above-described effect exhibited by the guide wire 100 according to the first embodiment. Moreover, the guide wire 100c according to the fourth embodiment further has the radiation-transmissible portion P0 as another part of the guide wire 100c in the longitudinal direction, which is located between the first radiopaque portion P1 and the second radiopaque portion P2 and free from radiopaque materials. Thereby, in the guide wire 100c according to the fourth embodiment, the first radiopaque portion P1 and the second radiopaque portion P2 can be more clearly distinguished from each other by the presence of the radiation-transmissible portion P0 free from radiopaque materials. Thus, the guide wire 100c according to the fourth embodiment can more reliably achieve both comprehension of the distal end position of the guide wire 100c by the presence of the first radiopaque portion P1 and comprehension of the blood vessel path by the presence of the second radiopaque portion P2. The guide wire 100c according to the fourth embodiment makes it possible to more reliably prevent the rotor RB supported by the second radiopaque portion P2 from coming into contact with the distal helical body 20.

### E. Fifth Embodiment:

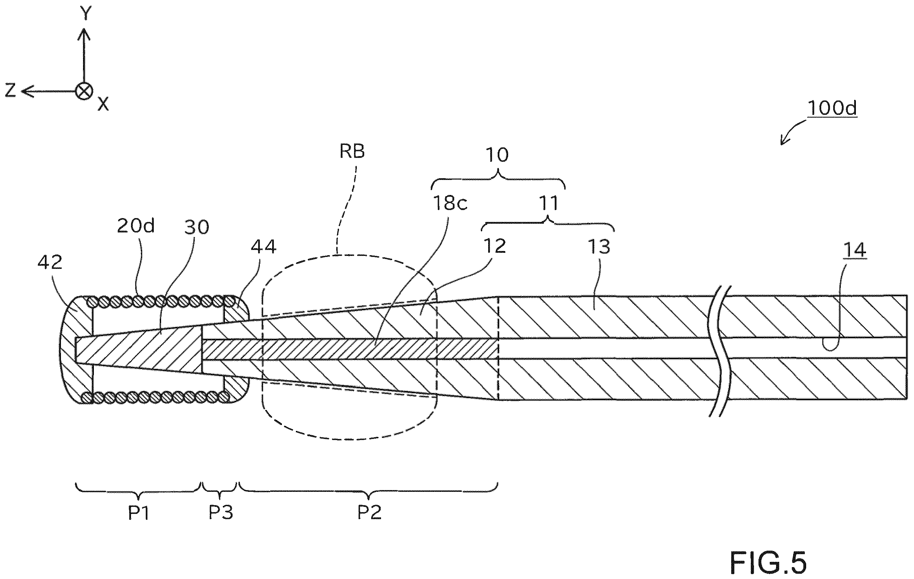

FIG. 5 is an explanatory diagram schematically illustrating a configuration of a guide wire 100d according to the fifth embodiment. In the description below, components of the guide wire 100d according to the fifth embodiment, which are the same as the above-described components of the guide wire 100 according to the first embodiment are given the same reference signs as in the first embodiment so as to omit the description of the same components in the fifth embodiment as appropriate.

The guide wire 100d according to the fifth embodiment includes a distal helical body 20d having a structure different from that of the guide wire 100 according to the first embodiment. Specifically, the guide wire 100d according to the fifth embodiment is configured such that the distal helical body 20d covers not only the entire distal core member 30 but also the distal end portion of the shaft body 10. That means, the distal helical body 20d has a length larger than of the distal core member 30, and the proximal end-side joint part 44 joins the proximal end of the distal helical body 20d with a portion at a more proximal position relative to the distal end of the shaft body 10.

Since the guide wire 100d according to the fifth embodiment is configured as described above, the guide wire 100d further has a third radiopaque portion P3 as another part of the guide wire 100d in the longitudinal direction, which is located between the first radiopaque portion P1 and the second radiopaque portion P2 and contains a radiopaque material. The first radiopaque portion P1 includes at least a part of the distal helical body 20d. In the fifth embodiment, the first radiopaque portion P1 is composed of a part on the distal end side of the distal helical body 20d and the entire distal core member 30. The second radiopaque portion P2 is located on the more proximal end side relative to the first radiopaque portion P1 and includes at least a part of the radiopaque core member 18 of the shaft body 10. In the fifth embodiment, the second radiopaque portion P2 is composed of the radiopaque core member 18 and a part on the proximal end side of the tapered portion 12 of the perforated shaft 11. The third radiopaque portion P3 is located between the first radiopaque portion P1 and the second radiopaque portion P2. In the fifth embodiment, the third radiopaque portion P3 is composed of a part on the proximal end side of the distal helical body 20d, and the radiopaque core member 18 and a part on the distal end side of the tapered portion 12 of the perforated shaft 11. In the fifth embodiment, the first radiopaque portion P1 is more radiopaque than the second radiopaque portion P2, and the third radiopaque portion P3 is more radiopaque than the first radiopaque portion P1.

Since the guide wire 100d according to the fifth embodiment has the same structure as the above-described structure of the guide wire 100 according to the first embodiment, the guide wire 100d exhibits the same effect as the above-described effect exhibited by the guide wire 100 according to the first embodiment. Moreover, the guide wire 100d according to the fifth embodiment further has the third radiopaque portion P3 as another part of the guide wire 100d in the longitudinal direction, which is located between the first radiopaque portion P1 and the second radiopaque portion P2 and contains a radiopaque material. The first radiopaque portion P1 is more radiopaque than the second radiopaque portion P2, and the third radiopaque portion P3 is more radiopaque than the first radiopaque portion P1. Thereby, in the guide wire 100d according to the fifth embodiment, the first radiopaque portion P1 and the second radiopaque portion P2 can be more clearly distinguished from each other by the presence of the third radiopaque portion P3 that is the most radiopaque. Thus, the guide wire 100d according to the fifth embodiment can more reliably achieve both comprehension of the distal end position of the guide wire 100d by the presence of the first radiopaque portion P1 and comprehension of the blood vessel path by the presence of the second radiopaque portion P2. The guide wire 100d according to the fifth embodiment makes it possible to more reliably prevent the rotor RB supported by the second radiopaque portion P2 from coming into contact with the distal helical body 20d.

### F. Sixth Embodiment:

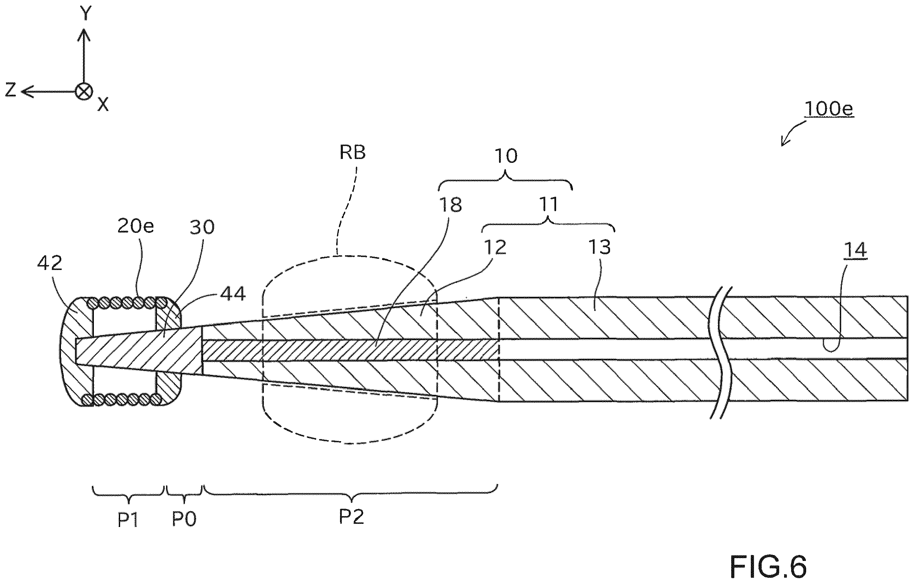

FIG. 6 is an explanatory diagram schematically illustrating a configuration of a guide wire 100e according to the sixth embodiment. In the description below, components of the guide wire 100e according to the sixth embodiment, which are the same as the above-described components of the guide wire 100 according to the first embodiment are given the same reference signs as in the first embodiment so as to omit the description of the same components in the sixth embodiment as appropriate.

The guide wire 100e according to the sixth embodiment includes a distal helical body 20e having a structure different from that of the guide wire 100 according to the first embodiment. Specifically, the guide wire 100e according to the sixth embodiment is configured such that the distal helical body 20e covers only a part on the distal end side of the distal core member 30 rather than the entire distal core member 30. That means, the distal helical body 20e has a length smaller than of the distal core member 30, and the proximal end-side joint part 44 joins the proximal end of the distal helical body 20e with a portion on a more distal end side relative to the proximal end of the distal core member 30.

Since the guide wire 100e according to the sixth embodiment is configured as described above, the first radiopaque portion P1 is composed of the entire distal helical body 20e and a part on the distal end side of the distal core member 30. Thus, the guide wire 100e according to the sixth embodiment has the radiation-transmissible portion P0 as another part of the guide wire 100e in the longitudinal direction, which is located between the first radiopaque portion P1 and the second radiopaque portion P2 and free from radiopaque materials. In the sixth embodiment, the radiation-transmissible portion P0 is composed of the proximal end portion (not covered with the distal helical body 20e) of the distal core member 30.

Since the guide wire 100e according to the sixth embodiment has the same structure as the above-described structure of the guide wire 100 according to the first embodiment, the guide wire 100e exhibits the same effect as the above-described effect exhibited by the guide wire 100 according to the first embodiment. Moreover, the guide wire 100e according to the sixth embodiment further has the radiation-transmissible portion P0 as another part of the guide wire 100e in the longitudinal direction, which is located between the first radiopaque portion P1 and the second radiopaque portion P2 and free from radiopaque materials. Thereby, in the guide wire 100e according to the sixth embodiment, the first radiopaque portion P1 and the second radiopaque portion P2 can be more clearly distinguished from each other by the presence of the radiation-transmissible portion P0 free from radiopaque materials. Thus, the guide wire 100e according to the sixth embodiment can more reliably achieve both comprehension of the distal end position of the guide wire 100e by the presence of the first radiopaque portion P1 and comprehension of the blood vessel path by the presence of the second radiopaque portion P2. The guide wire 100e according to the sixth embodiment makes it possible to more reliably prevent the rotor RB supported by the second radiopaque portion P2 from coming into contact with the distal helical body 20e.

### G. Modifications:

The technique disclosed in the present specification is not limited to the above-described embodiment, and various modifications can be made within the scope that does not depart from the gist thereof. For example, the following modifications can be made.

The configuration of the guide wire 100 according to each of the above-described embodiments is merely an example and may be modified in various ways. For example, although the perforated shaft 11 constituting the shaft body 10 is composed of the large diameter portion 13 and the tapered portion 12 in each of the embodiments described above, the perforated shaft 11 need not have at least one of these two portions, or may have another portion in addition to the two portions. For example, the perforated shaft 11 may further have a small diameter portion located on a more distal end side relative to the tapered portion 12 and having the same diameter as of the smallest diameter of the tapered portion 12. Alternatively, the perforated shaft 11 may further have a second tapered portion located on a more proximal end side relative to the large diameter portion 13 and having an outer diameter gradually increasing from a boundary position with the large diameter portion 13 toward the proximal end side, and a second large diameter portion located on a more proximal end side relative to the second tapered portion and having the same diameter as the largest diameter of the second tapered portion.

Although the radiopaque core member 18 is located throughout a portion formed on the tapered portion 12 within the hole 14 formed in the perforated shaft 11 in each of the above embodiments (except the fourth embodiment), the radiopaque core member 18 may be located only on a part formed on the tapered portion 12 within the hole 14. Although the radiopaque core member 18 is accommodated in a part formed on the tapered portion 12 within the hole 14 formed in the perforated shaft 11 in each of the above embodiments, the radiopaque core member 18 may be accommodated in a part formed on a portion (e.g., large diameter portion 13) other than the tapered portion 12 of the perforated shaft 11 within the hole 14.

Although the radiation-transmissible core member 19 is located throughout the portion formed on the large diameter portion 13 within the hole 14 formed in the perforated shaft 11 in the second embodiment, the radiation-transmissible core member 19 may be located only on a part of the portion formed on the large diameter portion 13 within the hole 14.

Although the perforated shaft 11 having the hole 14b as a bottomed hole is prepared by perforating the distal end side of the wire in the third embodiment, a perforated shaft having a bottomed hole may be prepared by forming a through-hole in the wire and then applying a brazing material or a resin member on the proximal end of the wire to close the proximal end side of the through-hole.

Although there is the gap 15 between the radiopaque core member 18c and the distal core member 30 in the longitudinal direction in the fourth embodiment, other members free from radiopaque materials may be located on at least a part of the gap 15. Even in such a configuration, there is a radiation-transmissible portion P0 free from radiopaque materials between the first radiopaque portion P1 and the second radiopaque portion P2.

Although the shaft body 10 is composed of the perforated shaft 11 and the radiopaque core member 18 in each of the above embodiments, the shaft body 10 may be composed of other components as long as the shaft body 10 has a portion containing a radiopaque material (specific portion). For example, the shaft body 10 may be configured such that a part of the shaft body 10 in the longitudinal direction contains a radiopaque material thoroughly from the center to the outer periphery.

Although the distal helical body 20 is composed of a coil body prepared by densely winding a strand in each of the above embodiments, the distal helical body 20 may be composed of a coil body prepared by coarsely winding a strand. Although the distal helical body 20 is composed of a coil body formed into a hollow cylindrical shape by spirally winding one strand in each of the above embodiments, the distal helical body 20 may be composed of a coil body formed into a hollow cylindrical shape by spirally winding a plurality of strands, or may be composed of a coil body formed into a hollow cylindrical shape by spirally winding one twisted wire formed by twisting a plurality of strands, or may be composed of a coil body formed into a hollow cylindrical shape by spirally winding a plurality of twisted wires formed by twisting a plurality of strands. The distal helical body 20 may be composed of a member other than the coil body, as long as the distal helical body 20 is formed into a spiral shape. For example, the distal helical body 20 may be composed of a member prepared by slitting a hypotube in a spiral shape.

Although the first radiopaque portion P1 is more radiopaque than the second radiopaque portion P2 in each of the above embodiments, conversely the first radiopaque portion P1 may be less radiopaque than the second radiopaque portion P2. Although the second radiopaque portion P2 has a length larger than of the first radiopaque portion P1 in each of the above embodiments, the second radiopaque portion P2 may have a length smaller than or equal to the length of the first radiopaque portion P1.

The material of each member according to each of the above embodiments is merely an example and may be modified in various ways. The manufacturing method of the guide wire 100 according to each of the above embodiments is merely an example and may be modified in various ways. For example, although the shaft body 10 is manufactured by inserting the radiopaque core member 18 into the hole 14 of the perforated shaft 11 prepared using a hypotube in each of the above embodiments, the shaft body 10 may be manufactured using a clad material having a core portion composed of a radiopaque material.

Although each of the above embodiments has been explained with reference to the guide wire 100 for atherectomy, the technology disclosed in the present specification can be similarly applied to guide wires for other uses.

### DESCRIPTION OF REFERENCE NUMERALS

10 Shaft body
11 Perforated shaft
12 Tapered portion
13 Large diameter portion
14 Hole
15 Gap
18 Radiopaque core member
19 Radiation-transmissible core member
20 Distal helical body
30 Distal core member
42 Distal end-side joint part
44 Proximal end-side joint part
100 Guide wire
P0 Radiation-transmissible portion
P1 First radiopaque portion
P2 Second radiopaque portion
P3 Third radiopaque portion
RB Rotor

## Claims

1. A guide wire comprising:
a shaft body having a specific portion containing a radiopaque material; and
a distal helical body formed into a spiral shape, which contains a radiopaque material and has at least a part located on a more distal end side relative to the shaft body, wherein
the guide wire includes:
a first radiopaque portion as a part of the guide wire in a longitudinal direction of the guide wire, which includes at least a part of the distal helical body; and
a second radiopaque portion as another part of the guide wire in the longitudinal direction of the guide wire, which is located on a more proximal end side relative to the first radiopaque portion, includes at least a part of the specific portion of the shaft body, and has a radiopacity different from that of the first radiopaque portion.

2. The guide wire according to claim 1, wherein
the first radiopaque portion is more radiopaque than the second radiopaque portion.

3. The guide wire according to claim 1 or 2, wherein
the second radiopaque portion has a length larger than that of the first radiopaque portion in the longitudinal direction of the guide wire.

4. The guide wire according to any one of claims 1 to 3, further including
a radiation-transmissible portion as another part of the guide wire in the longitudinal direction of the guide wire, which is located between the first radiopaque portion and the second radiopaque portion and free from radiopaque materials.

5. The guide wire according to any one of claims 1 to 4, further including
a third radiopaque portion as another part of the guide wire in the longitudinal direction of the guide wire, which is located between the first radiopaque portion and the second radiopaque portion, and contains a radiopaque material, wherein
the first radiopaque portion is more radiopaque than the second radiopaque portion; and
the third radiopaque portion is more radiopaque than the first radiopaque portion.

6. The guide wire according to any one of claims 1 to 5, wherein
the shaft body includes:
a perforated shaft having a hole that opens on a distal end of the perforated shaft and extends in a longitudinal direction of the perforated shaft; and
a first core member accommodated in the hole and serving as the specific portion containing the radiopaque material.

7. The guide wire according to claim 6, wherein
within the hole formed in the perforated shaft, a portion on a more proximal end side relative to a portion accommodating the first core member is hollow.

8. The guide wire according to claim 6, wherein
the shaft body further includes a second core member accommodated in a portion on the more proximal end side relative to a portion accommodating the first core member within the hole formed in the perforated shaft, the second core member being free from radiopaque materials.

9. The guide wire according to claim 6, wherein
the hole formed in the perforated shaft is a bottomed hole having a bottom face at a more distal position relative to a proximal end of the perforated shaft.

10. The guide wire according to any one of claims 6 to 9, wherein
in the second radiopaque portion, the perforated shaft has a stiffness higher than that of the first core member.

11. The guide wire according to any one of claims 1 to 10, wherein
the guide wire is an atherectomy guide wire rotatably supporting a rotor disposed in an atherectomy catheter on the second radiopaque portion.
